# EUROPEAN PATENT SPECIFICATION

(11) **EP 4 097 732 B1**
(45) Date of publication and mention of the grant of the patent: **04.06.2025**
(21) Application number: 21747798.3
(22) Date of filing: 29.01.2021
(51) Int. Cl.: G16H 10/60, G16H 80/00, G16H 40/67

(54) **HEALTHCARE INFORMATION NETWORK OF NETWORKS**
GESUNDHEITSINFORMATIONSNETZWERK FÜR NETZWERKE
RÉSEAU DE RÉSEAUX D'INFORMATIONS DE SOINS DE SANTÉ

(30) Priority: 29.01.2020 US 202062967448 P; 03.02.2020 US 202062969564 P
(43) Date of publication of application: 07.12.2022
(73) Proprietor: CORANET SOLUTIONS, INC., Washington, DC 20006 (US); Alisuag, Cora, Washington, DC 20008 (US)
(72) Inventor: ALISUAG, Cora, Washington, DC 20008 (US)
(74) Representative: Patentanwälte Dr. Keller, Schwertfeger Partnerschaft mbB
(86) International application number: PCT/US2021/015934
(87) International publication number: WO 2021/155313

(56) References cited:
- CA-A1- 2 342 977
- CA-A1- 2 342 977
- US-A1- 2001 044 823
- US-A1- 2001 044 823
- US-A1- 2010 205 597
- US-A1- 2010 205 597
- US-A1- 2011 301 981
- US-A1- 2011 301 981

## Description

### FIELD OF THE INVENTION

This disclosure relates generally to a mobile personal health information exchange. More specifically, the mobile personal health information exchange provides an electronic health record aggregated from multiple sources of health records, to which access is controlled by the user, or patient, and which is updated automatically.

### BACKGROUND

Facing challenges in quality of care and runaway costs, resulting in unsustainable fiscal challenges, healthcare systems are under a financial burden. Without mitigation, the continued growth of this burden ultimately leads to higher costs or lower quality care to the patient. Lessening this burden, while improving quality of care, in many instances, may be as simple as collecting a complete medical history from or for an individual. When an individual sees a healthcare provider under normal circumstance, such as for a scheduled appointment to treat an illness, a complete medical history is collected before rendering care. The value of obtaining a complete medical history, and the cost savings it provides, cannot be understated. More valuable in diagnosis than laboratory tests, seventy-six percent (76%) of diagnoses result from medical histories compared to eleven percent (11%) from laboratory test and twelve percent (12%) from physical examination. (Peterson et al., "Contributions of the history, physical examination, and laboratory investigation in making medical diagnoses," Western Journal of Medicine, 1992 Feb; 156(2): 163-165.) Filling out forms may seem annoying and time consuming, but it allows healthcare providers to make accurate diagnoses without relying on costly tests and examinations.

A complete medical history would ideally include all diagnoses, testing, and treatment records from all sources of medical treatment, including ambulatory medical services, emergency medical services, and routine doctor visits for check-ups or minor illnesses. It would also be desirable to include radiological and other images, results from any laboratory testing, and video and audio from telemedicine services. A growing market in medical patient monitors allows a patient to monitor a variety of real-time biometric and/or medical data such as blood pressure, heart rate, electrophysiology, body temperature, blood oxygen saturation, posture, physical activities, and various sweating biomarkers. The ability to process and add such real-time data to an electronic medical history would further improve the usefulness of a complete medical record. Ideally, processing of such real-time data would include a comparison to standardized medical data including expected values of measured characteristics for the patient's demographic group and identification of any possible health-related abnormalities.

In addition to having a complete medical history for a patient available, it would further be desirable for such medical history to be updated automatically with no affirmative action required by the patient. "As widespread adoption of the electronic health record (EHR) takes place in US medical practice, many health care experts have emphasized the promising capabilities of the EHR to foster patient activation, which is a characteristic of patients who view themselves as active collaborators in their own health care management.... After reviewing their visit notes, patients reported feeling more in control of their care." (White, Amina, and Marion Danis. "Enhancing patient-centered communication and collaboration by using the electronic health record in the examination room." JAMA vol. 309, 22 (2013): 2327-8. doi: 10.1001/ jama.2013.6030.) Automated availability of complete, up-to-date health records not only reduces cost and effort for all involved in the health care process but promotes better long-term health through the patient's review and control of these records.

US 2010/205597 A1 discloses a method of managing healthcare data. Healthcare data of the first healthcare data base operated by a first healthcare data management system (HDMS) is collected. The collected healthcare data is stored in a second healthcare database operated by the second HDMS. The healthcare data between the first healthcare database and the second healthcare database are synchronized by detecting a change in the healthcare data of the first healthcare database, creating a delta file representing the change in the healthcare data of the first healthcare database, transmitting the delta file to the second HDMS and updating the healthcare data of the second healthcare database based on the delta file. This document fails to disclose a network of patient monitors configured to communicate with one or more of the health organization networks via the central service provider through the communication link. This document can be considered to be the closest prior art.

### BRIEF DESCRIPTION OF THE DRAWINGS

The present disclosure is illustrated by way of example, and not by way of limitation, in the accompanying drawings, in which like reference characters refer to like parts throughout, and in which:

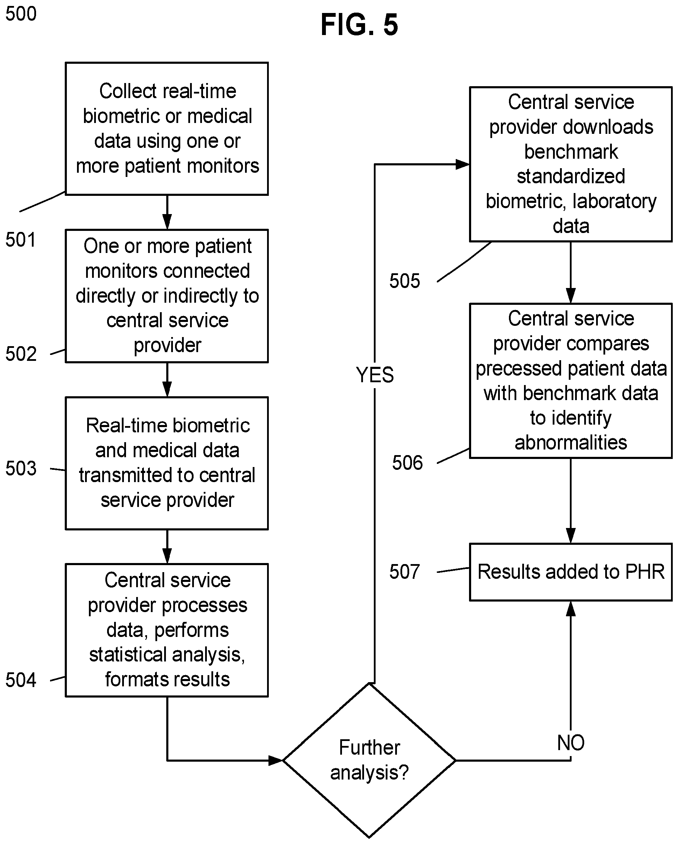

FIG. **1** shows an architecture of system comprising a network of networks. Descriptions of FIG. **2**-FIG. **5** in the DETAILED DESCRIPTION refer to reference numbers in FIG. **1** to provide context for the flow of electronic health record information in each process represented.
FIG. **2** is a flow diagram for updating electronic health records in a personal health information exchange system applied in a personal repository.
FIG. **3** is a flow diagram for updating electronic health records in a personal health information exchange system related to delivery of medical services.
FIG. **4** is a flow diagram for updating electronic health records in a personal health information exchange system related to emergency medical services.
FIG. **5** is a flow diagram for updating electronic health records in a personal health information exchange system related to use of patient monitors.

### DETAILED DESCRIPTION

Various aspects will now be described with reference to specific forms selected for purposes of illustration.

The words and phrases used herein should be understood and interpreted to have a meaning consistent with the understanding of those words and phrases by those skilled in the relevant art. No special definition of a term or phrase, i.e., a definition that is different from the ordinary and customary meaning as understood by those skilled in the art, is intended to be implied by consistent usage of the term or phrase herein. To the extent that a term or phrase is intended to have a special meaning, i.e., a meaning other than the broadest meaning understood by skilled artisans, such a special or clarifying definition will be expressly set forth in the specification in a definitional manner that provides the special or clarifying definition for the term or phrase.

For example, the following discussion contains a non-exhaustive list of definitions of several specific terms used in this disclosure (other terms may be defined or clarified in a definitional manner elsewhere herein). These definitions are intended to clarify the meanings of the terms used herein. It is believed that the terms are used in a manner consistent with their ordinary meaning, but the definitions are nonetheless specified here for clarity.

Each of the following terms written in singular grammatical form: "a," "an," and "the," as used herein, may also refer to, and encompass, a plurality of the stated entity or object, unless otherwise specifically defined or stated herein, or, unless the context clearly dictates otherwise.

### Types of Records

"Electronic medical record" (EMR) typically refers to a digital version of a patient's charts created by individual health facilities or providers. Unlike paper charts, EMRs can help clinicians track and monitor data over time and identify patients with particular needs. The biggest drawbacks to EMRs is that they are kept by individual providers of medical services and do not typically support interoperability or the exchange of information with other providers. Although a digitally stored EMR is an improvement over traditional paper charts, its usefulness is limited by a lack of standards-based interoperable data. An EMR is generally a patient's record associated with a specific provider.

"Electronic health record" (EHR), like an EMR, is a digital version of a patient's chart. An EHR, however, differs from an EMR in that it includes data from all medical service providers and facilities involved in a patient's care and participating in the relevant EHR system. All authorized providers and staff can access information in an EHR to treat a patient. An EHR can also include information about: diagnoses, allergies, medications, treatment plans, immunization dates, test results (including radiology and other images). Ideally, an EHR is complete medical history associated with a patient and is not specific to a specific provider. For example, a patient receiving care for a back condition might receive care from a primary care physician, an orthopedic specialist, a magnetic resonance imaging (MRI) facility, and a physical therapist. Each of these providers would contribute information about the patient's treatment to the same EHR. This makes the EHR essential for data sharing and coordination of care. As widespread adoption of the EHR takes place in U.S. medical practice, many health care experts have emphasized the promising capabilities of the EHR to foster active participation of the patient in managing their own health care.

"Personal health record" (PHR), like an EMR or EHR is likely maintained digitally but unlike the EMR or EHR is controlled by the patient instead of the medical service provider(s). Data kept within a PHR may come from an EHR, but the patient is in charge of management of and access to the PHR. A patient can enter information into their PHR themselves, such as their lifestyle habits, over-the-counter medications, home monitoring devices, wearables, biometrics devices, and additional care providers that may not be part of an EHR system.

As used herein, "PHR" will refer to the patient's EHR stored on the patient's personal repository, and EHR refers to the patient's health history stored in the repository of one or more medical service providers. One aspect of the invention disclosed herein is that although the patient controls the PHR, the patient authorizes synchronization of the PHR and EHR within the system such that the PHR is automatically and periodically updated to reflect a complete medical history and the EHRs of authorized providers will contain the same updated information.

### Sources of Medical Data

"Ambulatory medical services," as used herein, means medical services performed on an outpatient basis, without admission to a hospital or other facility. Ambulatory patient services include, but are not limited to, visits to specialists, physical therapy, trips to emergency room when the patient is mobile., diagnostic tests, treatments, or rehabilitation visits. The location at which ambulatory services can be delivered includes doctor's office, clinic, emergency room, and outpatient hospital department. Nonlimiting examples of ambulatory care include blood tests, biopsy, chemotherapy, colonoscopy, CT scan, mammograms, minor surgical procedures, radiation treatments, ultrasound imaging, and x-rays.

"Emergency medical services," as used herein, means provision of medical services to the patient when the patient is suffering from a medical condition manifesting itself by acute symptoms of severity (including severe pain), such that a prudent layperson, who possesses an average knowledge of health and medicine, could reasonably expect the absence of immediate medical attention to result in placing the health of the individual in serious jeopardy. Such medical conditions requiring emergency services include natural events, such as a heart attack, diabetic shock, or traumatic events, such as an automobile accident, stabbing, or gunshot wound. In either case, an aspect of emergency services provided in the context of the personal health information exchange system is that the event precipitating the need for emergency services occurs at a location distant from medical service providers, such as when the patient is at home, at work, or even on vacation distant from home. Additionally, the patient requiring emergency services may be unconscious or otherwise incapacitated, such that the patient is unable to assist in their own care.

"Telemedicine services," as used herein, means the remote diagnosis and treatment of patients by means of telecommunications technology. Telemedicine is the exchange of medical information from one location to another using electronic communication in order to improve the health status of a patient. Such electronic communication includes wireless tools, email, two-way video, smartphones, and other methods of telecommunications technology. As early as the 1980's, hospitals began extending their services to patients in remote locations. Since then, telemedicine has become an integrated part of specialty departments, hospitals, private doctor offices, and home health care. A patient can receive the benefits of telemedicine at home, at work, or even when traveling. The products and services of telemedicine are not a separate medical specialty but are instead part of the expanded platform of healthcare settings providing an alternative means for delivering healthcare. Billing and coding for remote medical services provided through telemedicine are typically the same or very similar to and those for equivalent services delivered on site.

"Routine medical services," as used herein, means traditional delivery of medical services for check-ups and/or diagnosis, testing, and treatment for minor illnesses (e.g. a normal visit to a primary care physician). Routine medical services can also include prescribing and filling prescriptions for medication either by a physician or pharmacist.

"Standardized medical data," as used herein, means a database maintained by a provider that contains the compiled biometric and medical data of a statistically significant number of people in a standardized format. The database can contain biometric and/or medical data such as, but not limited to, one or more of blood pressure, heart rate, electrophysiology, body temperature, blood oxygen saturation, posture, physical activities, and various sweating biomarkers. The data is categorized by one or more of height, weight, age, demographic data, geographical region, or other relevant factors and statistically processed to provide benchmark information for comparison to real-time biometric and/or medical data gathered from the patient in order to identify any possible health-related abnormalities.

### Components of the Personal Health Information Exchange

"Application Programming Interface" or "API," as used herein, is a software-to-software interface and not a user interface with software. An API is a set of commands, functions, protocols, and objects that can be used to interact with an external system. APIs are used to control access to resources such as hardware devices and software functions that an application may not necessarily have permission to use, which is why APIs often play a big role in security. APIs are also used for communication between services. With APIs, applications communicate without any user knowledge or intervention. The user sees only one interface, but behind the scenes, many applications may be working together using APIs. This type of integration is often called "seamless," since the user never notices when software functions are handed from one application to another. An API resembles Software as a Service (SaaS), since software developers do not have to start from scratch every time they write a program. Instead of building one core application that tries to do everything, the application can contract out certain responsibilities to remote software that already performs specific tasks efficiently. An API can be configured to maintaining a ledger associated with an EHR. In addition to maintaining the ledger, the API can be configured to update EHRs throughout all or a portion of the network of networks. For instance, when a participant makes a change to a medical history the API can send instructions directing medical histories stored in all or a portion of the network of networks be updated to reflect the change.

"Cartridge," as used herein, means software components that extend a server. Cartridge constituents reside on the server or are accessed from the server. Packaging domain-specific component expertise in a data cartridge allows the cartridge to access the corporate information repository and add both organizational and operational value to the data. Such software components are applications that can be "plugged" into other software components, and which are themselves "pluggable." Data cartridges are server-based, extend the server, are integrated with the server, are packaged to address access issues of various target users.

"CCD," as used herein, means Continuity of Care Document, which is based on the HL7 CDA architecture. The CCD is a joint effort of HL7 International and ASTM. CCD fosters interoperability of clinical data by allowing physicians to send electronic medical information to other providers without loss of meaning and enabling improvement of patient care. CCD is an implementation guide for sharing Continuity of Care Record (CCR) patient summary data using the HL7 Version 3 Clinical Document Architecture (CDA), Release 2. CCD establishes a rich set of templates representing the typical sections of a summary record and expresses these templates as constraints on CDA. These same templates for vital signs, family history, plan of care, and so on, can then be reused in other CDA document types, establishing interoperability across a wide range of clinical use cases. Some of its benefits are: allowing physicians to send electronic medical information to other providers without loss of meaning and provides a "snapshot in time," constraining a summary of the pertinent clinical, demographic, and administrative data for a specific patient.

"CDA," as used herein, means Clinical Document Architecture, which is a document standard governed by the HL7 organization. The HL7 CDA is a document markup standard that specifies the structure and semantics of "clinical documents" for the purpose of exchange. It supports exchange of human-readable documents between users, including those with different levels of technical sophistication.

"Central service provider," as used herein, is a connection point for multiple networks and can further host multiple APIs and/or cartridges to facilitate communication between the networks and perform processing on data exchanged between networks. A preferred central service provider is configured to enable authenticated access to one or more medical EHR systems. For instance, a central service provided can be configured to store profiles for various networks, healthcare providers, and/or individuals and to verify ownership of a profile using one more of personal identification numbers, passwords, biometrics and/or unique numbers. Once a network is verified, a central service provider can be configured to provide an approval code enabling establishment of a communication link between the requesting and transmitting networks, enabling transmission of the medical history. In some embodiments, the central service provider does not require purchase of an information technology (IT) product by a user or organization, but instead builds, operates, and manages IT products, which are bundled and delivered as a service/solution. A customer can access this type of service/solution from a central service provider via several different sourcing models, including, but not limited to, a monthly or annual subscription fee.

"Communication link," as used herein, means any form of electronic connection suitable for the secure transmission of data between a health organization network and a central service provider. Communication links are not particularly limited and can include any communication channel through which data can be transmitted. For instance, communication links can comprise a direct connection through telephone lines, satellite links, radio transmissions digital subscriber line, internet, cellular networks, cloud-based communications and/or other transmission channels, or an indirect connection which can be any combination of the foregoing. Examples of security protocols for these links include, but are not limited to, Secure Electronic Transactions (SET), Secure Socket Layer (SSL), Secure Hypertext Transfer Protocol (S-HTTP), Secure Shell (SSH-2), and IP Security (IPSec). Communication links can comprise Transport Layer Security (TLS 1.2 &TLS 1.3) protocol for encrypted transmissions of EHR data. A preferred communication link protocol can be two-way TLS, also known as TLS with client certificate authentication. A preferred configuration would utilize 2048-bit SSL certificates along with - 256-bit encryption.

"Emergency Health Information Exchange" network or "EHIE" means a health organization network, which is part of the personal health information exchange network used by health organizations for the purpose of exchanging personal health information needed in critical, emergency situations and which can be accessed without physical action by the patient. All or a portion of the patient's EHR is maintained on the EHIE. In any event, the portion of the EHR available through the EHIE is at least enough for first responders to provide standard care required prior to transport of the patient to an emergency room at a hospital or other similar facility. Despite the convenience and access to data personal repositories provide, often in emergency and/or disaster situations, a user may be unable to access a personal repository. For instance, a user may be unconscious and/or without cellular service. In such instances, healthcare providers may need to access at least a portion of the user's EHR, such as lifesaving information only (name, date of birth, gender, blood type, allergies, and current medications. Through communication link associated with the EHIE, first responders and/or emergency room personnel can obtain a victim's EHR as pre-authorized by the user. To facilitate obtaining information from an unresponsive user and/or a user without that ability to access their personal repository, the EHIE can comprise a communication link to an identification repository. For instance, the identification repository can comprise a National Master Patient Index and/or driver registry. The link can be configured to enable the transmissions of user identifying information such as, but not limited to, a photograph of victim, fingerprint, biometric data, and/or other data uniquely identifying an individual. Once identified, all or a portion of the user's EHR, as pre-authorized by the user, can be obtained through the network of networks.

"Health organization network," as used herein, means a linkage of files or servers within one organization or a linkage of multiple organizations using a common platform for storing and accessing EHRs. Transmission of EHRs between health organization networks requires files in CCD format or additional APIs residing on the central service provider linking the networks to facilitate translation. Health organization networks can comprise a network of multiple clinics, offices, hospitals, and/or other facilities managing EHRs. A health organization network can also be an individual provider of ambulatory medical services, emergency medical services, telemedicine services, or routine medical services. For instance, one or more of health organization networks can comprise a clinic housing the medical histories of its patients on a single local server. It also possible one or more of health organization networks can comprise cloud storage holding medical histories. Regardless of complexity, size and manner in which EHRs are stored, each health organization network contains one or more repositories of EHRs. By communicating via communication links through central service provider, health organization networks form a network of networks enabling the exchange of medical histories. Regardless of communication channels employed, health organization networks comprise repositories of EHRs, and during the course of providing medical care, these EHRs are updated. The EHIE is a specific type of health organization network.

"HL7," as used herein, means a standard for exchanging information between medical applications. It defines a format for the transmission of health-related information. HL7 is an international standard used to transfer and share data between various healthcare providers and helps bridge the gap between health information technology applications by allowing easier and more efficient sharing of healthcare data. HL7 Fast Healthcare Interoperability Resources (FHIR^{™}) refers to an interoperability standard facilitating the exchange of healthcare information between healthcare providers, patients, caregivers, payers, researchers, and any other party involved in the healthcare system. HL7 FHIR^{™} represents granular clinical concepts which can be used to exchange to quickly and effectively solve problems in healthcare and related process and includes the basic elements of healthcare, like patients, diagnostic reports, admissions, medications, and problem lists, along with their typical participants. HL7 FHIR^{™} can also support a range of more complex clinical models. Resources are generally based on thorough requirements gathering, formal analysis, and extensive cross-mapping to other relevant standards.

"Ledger," as used herein, means a transaction record related to an EHR. Distributed ledger technology is the use of multiple ledgers that are synchronized to provide synchronized EHRs and PHR. A ledger comprises a system of codes detailing one or more of the origin of a transfer of an EHR, the destination of the transfer of an EHR, changes made to an EHR, the origin of change to an EHR, and/or route of transmission. The codes can comprise institution code, a state and/or country code, a location code, and/or a branch code. Regardless of the communication link, each network within a peer-to-peer network will maintain ledgers reflecting changes to the medical histories. Similar to the common use of the word ledger, a ledger in the context of Distributed Ledger Technology is simply an electronic record or collection of transactions. An important distinction is that the ledger is not a collection of assets. While assets are a part of transactions, the ledger records the transactions related to those assets.

"Master patient index," as used herein, means a form of customer data integration (CDI) specific to the healthcare industry. Healthcare organizations and groups use Enterprise Master Patient Index (EMPI) to identify, match, merge, de-duplicate, and cleanse patient records to create a master index that may be used to obtain a complete and single view of a patient.

"Patient," "user," and "victim" are used interchangeably herein.

"Patient monitors," as used herein, means devices, often using wearable technology, suited for monitoring a variety of a patient's real-time biometric and/or medical data such as, but not limited to, blood pressure, heart rate, electrophysiology, body temperature, blood oxygen saturation, posture, physical activities, stress level, EKG patterns, weight tracking, and various sweating biomarkers. Data from such patient monitors can be collected for analysis by storage on the patient monitor for later download, transmitted to the patient's mobile device, such as but not limited to a smartphone, or transmitted directly to a service associated with a particular patient monitor. In any case, there will be software, associated with the device, tailored for processing the specific type of biometric and/or medical data being collected. In conjunction with the collection of real-time biometric and/or medical data from the patient, standardized medical data can be accessed for comparison of the patient's real-time biometric and/or medical data to the expected real-time biometric and/or medical data for the patient to identify any possible health-related abnormalities.

"Personal repository," as used herein, means any device having a storage medium and user interface permitting the medical history to be display. Accordingly, personal repositories can comprise smartphones, cell phones, laptops, desktop computers and/or patient monitors. Regardless of the specific form, personal repositories enable an individual to share their PHR or EHR with a healthcare provider.

"Providers of medical services," as used herein, means entities engaged in providing ambulatory medical services, emergency medical services, telemedicine services, or routine medical services, and that also maintain EHRs for customers/patients.

"Server enhancement," as used herein, means one or more APIs, one or more cartridges, or combinations thereof.

### Description of System Embodiment

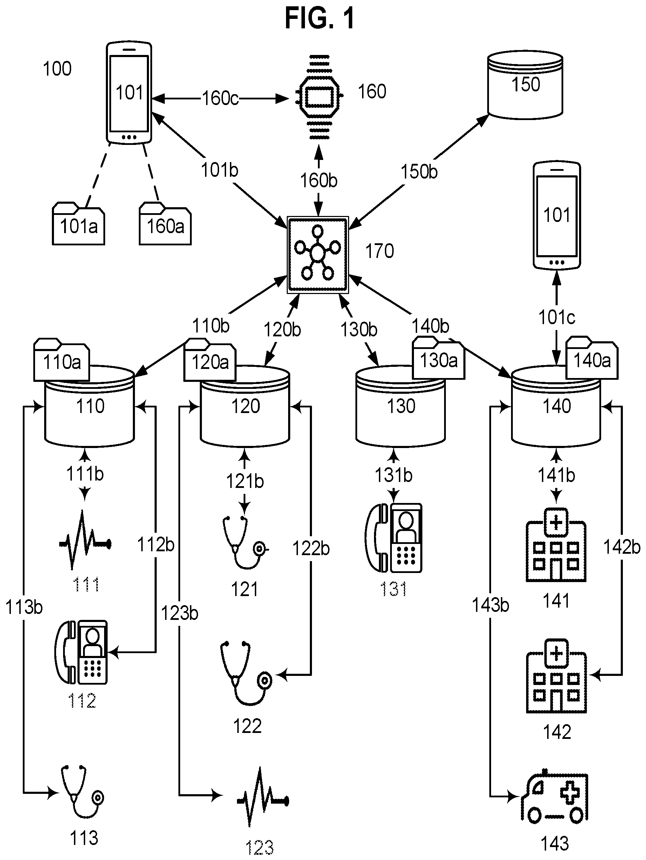

Providing seamless sharing of medical histories, the architecture comprises a central service provider **170,** as shown in **FIG. 1****.** Configured to operate as gateway, central service provider **170** establishes communication links **110b, 120b,** and **130b** between different health organization networks **110, 120,** and **130,** respectively.

In addition to health organization networks **110, 120,** and **130,** the architecture can comprise a network of patient monitors **160** communicating with one or more of the health organization networks **110, 120,** and **130** via central service provider **170** through one or more communication links **110b, 120b,** and **130,** respectively. All or a portion of the patient monitors **160** can utilize a computer, such as a cell phone or laptop, to communicate with central service provider **101.** Regardless of whether patient monitors communicate with service provider **101** directly or indirectly, patient monitors **160** collect biometric and/or other medical data. Transmitting the collected data through service provider **170** to all or a portion of health organization networks **110, 120,** and **130,** patient monitors **104** update a medical history.

The network of networks further comprises one or more personal repositories **101** storing medical histories. Through communication link **102,** personal repositories **101** can obtain and store one or more medical histories.

For when a user is unresponsive and/or lacks the ability to access the personal repository, the network of networks can further comprise an EHIE network **140.** Through communication link **140b,** emergency medical services providers can obtain all or a portion of the user's medical history. Additionally, the EHIE network **140** can comprise a link to an identification repository to aid in identifying the incapacitated user.

Accuracy of the medical records obtained through the network of networks is essential. Maintaining ledgers of transactions on one of more networks **110, 120, 130,** and/or **140** can facilitate such accuracy. Accordingly, the architecture and/or system can comprise one or more ledgers stored within one or more of networks **110, 120, 130,** and/or **140.** As to facilitate maintaining the ledgers in a trusted state, all or a portion of networks **110, 120, 130,** and/or **140** can be organized as peer-to-peer networks and/or make use of cartridges and/or distributed ledger technology. Regardless of the organization of networks **110, 120, 130,** and/or **140,** central service provider **170** is configured to provide a consistent server enhancement for each network of the network of networks. Each network, ideally, but not necessarily, utilizes the same server enhancement to interface with central service provider **170.** The single and/or multiple server enhancements provided by central repository **170** can be configured to provide a private subnetwork between networks through all or a portion of communication links **170.**

Providing a communication link between networks, the central repository can be configured to only enable passing of information. Merely passing information between networks of the network of networks, central service provider **170** can be configured not to store medical histories after transmission to the receiving network. Accordingly, central service provider **170** can be configured to only transiently store information identifying a patient, document, and/or medical history necessary for entry into the ledger. Once the receiving and/or transmitting networks have verified successful updating of the ledgers, central service provider **170** can be configured to delete all or a portion of the transmitted medical history and/or accompanying ledger information.

### Description of System Method

In an embodiment shown in **FIG. 1****,** the focal point of the personal health information exchange (PHIE) system **100** is the personal repository **101,** which is an electronic mobile device, such as, but not limited to, a smartphone. The PHIE system **100** functions to aggregate and transmit to the personal repository **101** a comprehensive and current EHR, which is the context of the patient's personal repository is equivalent to and interchangeable with PHR **101a.** The PHIE system **100** functions to automatically update the PHR **101a** based on an update to the patient's EHR maintained by one or more health organization networks **110, 120, 130,** and/or **140,** which subscribes to the PHIE system **100.**

The user, or patient, is in control of the personal repository **101.** The personal repository **101** is connected to a central service provider **170** through a communication link **101b** related to the personal repository **101.** The central service provider **170,** in turn, connects to one or more health organization networks: health organization network **110,** which maintains a copy of patient's EHR **110a,** through a communication link **110b** related to health organization network **110;** health organization network **120,** which maintains a copy of patient's EHR **120a,** through a communication link **120b** related to health organization network **120;** health organization network **130,** which maintains a copy of patient's EHR **130a,** through a communication link **130b** related to health organization network **130;** and emergency health information exchange network **140,** which maintains a copy of all or a portion of a patient's EHR **140a,** through a communication link **140b** related to health organization network **140.**

In the embodiment of the PHIE **100** shown in **FIG. 1****,** health organization network **110,** in turn, is connected to one or more providers of medical services: an ambulatory services provider **111,** which maintains a copy of patient's EHR **111a,** through a communication link **111b** related to the ambulatory services provider **111;** a telemedicine services provider **112,** which maintains a copy of patient's EHR **112a,** through a communication link **112b** related to the telemedicine services provider **112;** and/or a routine medical services provider **113,** which maintains a copy of patient's EHR **113a,** through a communication link **113b** related to the routine medical services provider **113.**

In the embodiment of the PHIE **100** shown in **FIG. 1****,** health organization network **120,** in turn, is connected to one or more providers of medical services: a routine medical services provider **121,** which maintains a copy of patient's EHR **121a,** through a communication link **121b** related to the routine medical services provider **121;** a routine medical services provider **122,** which maintains a copy of patient's EHR **122a,** through a communication link **122b** related to the routine medical services provider **122;** and a telemedicine services provider **121,** which maintains a copy of patient's EHR **121a,** through a communication link **121b** related to the telemedicine services provider **121.**

In the embodiment of the PHIE **100** shown in **FIG. 1****,** health organization network **130,** in turn, is connected to a telemedicine services provider **131,** which maintains a copy of patient's EHR **131a,** through a communication link **131b** related to the routine medical services provider **131.** In this instance, telemedicine services provider **131** might not be part of a broader health organization network with other members. Health organization network **130,** in this case could be a network owned and operated by telemedicine services provider **131,** such that telemedicine services provider **131** is linked directly to the central services provider **170.**

In the embodiment of the PHIE **100** shown in **FIG. 1****,** health organization network **140,** in turn, is connected to one or more providers of emergency medical services: a hospital emergency room **141,** which maintains a copy of patient's EHR **141a,** through a communication link **141b** related to the hospital emergency room **141;** a hospital emergency room **142,** which maintains a copy of patient's EHR **142a,** through a communication link **142b** related to the hospital emergency room **142;** and a first responder services provider **143,** which maintains a copy of patient's EHR **143a,** through a communication link **143b** related to the first responder services provider **143.** If the patient is conscious and the patient personal repository 101 is available, providers of emergency medical services **141, 142, 143** can access critical patient information as previously authorized by patient (i.e. when subscribing to the PHIE) through communication link **101c.**

The above description are nonlimiting illustrations of the possible variations of medical services included in the PHIE disclosed herein. An individual health organization network can be a single provider of ambulatory, telemedicine, routine, or emergency services. Alternatively, an individual health organization network can comprise one or more providers of a single service type selected from ambulatory, telemedicine, routine, or emergency services. In other cases, an individual health organization network can comprise one or more providers, in any combination, selected from one or more of ambulatory, telemedicine, routine, and/or emergency services.

In some embodiments, each medical services provider is part of a different health organization network than each of the other medical services providers, in which case one or more APIs residing on the central service provider **170** will enable the connection between each medical services provider and the central service provider **170** by way of the communication link related to each such medical services provider to form a network of networks. The one or more APIs hosted by the central service provider **170** can be configured to provide a private subnetwork between networks through all or a portion of communication links **110b, 120b, 130b,** and/or-**140b.**

EHR and PHR information and updates are securely transmitted through the network of networks. Accuracy of the EHRs **110a, 120a, 130a,** and/or **140a** maintained by each health organization network **110, 120, 130,** and/or **140** is assured by one or more ledgers of transactions related to each EHR and PHR. The PHIE system **100** comprises one or more ledgers stored within one or more of networks of each provider of medical services **110, 120, 130,** and/or **140.** All or a portion of providers of medical services **110, 120, 130,** and/or **140** can be organized as peer-to-peer networks in order to maintain the ledgers in a trusted state. The central service provider **170** is configured to provide a consistent server enhancement for each provider of medical services **110, 120, 130,** and/or **140** joined in the network of networks. The one or more APIs residing on the central service provider **170** can further be configured to provide a private subnetwork between networks through all or a portion of communication links **110b, 120b, 130b,** and/or **140b.**

In an embodiment, the central service provider **170** is configured to transmit information. Acting as a gateway between networks of the providers of medical services **110, 120, 130,** and/or **140,** central service provider **170** is configured to store EHR information transiently after transmission to the receiving service provider and erased once the receiving and/or transmitting service providers have verified successful updating of the relevant ledgers of transactions.

Each provider of medical services **110, 120, 130,** and/or **140** within a peer-to-peer network will maintain ledgers reflecting changes to the EHRs **110a, 120a, 130a,** and/or **140a.** Besides maintaining the ledger, the one or more server enhancements are configured to update EHRs **110a, 120a, 130a,** and/or **140a,** always including the PHR **101a.** A preferred communication link protocol can be two-way TLS, also known as TLS with client certificate authentication. A preferred configuration would utilize 2048-bit SSL certificates along with 256-bit encryption.

In addition to connections to health organization networks **110, 120, 130,** and/or **140,** the PHIE system **100** can include one or more patient monitors **160** for collection of real-time biometric and medical data from the patient. A patient monitor can be connected to the PHIE system **100** through a direct connection to central service provider **170** by a communication link related to the patient monitor **160a.** Alternately, a patient monitor can be connected to the PHIE system **100** through an indirect connection to central service provider 170 by connection to the patient's personal repository **101** through evolving wireless means, including but not limited to Bluetooth^{™} technology, which in turn is connected to central service provider **170** by a communication link related to the patient's personal repository **101.** Depending on applications available on the personal repository **101,** the personal repository **101** can act as a gateway to stream the real-time biometric and medical data from the patient to the central service provider **170,** or the real-time biometric and medical data from the patient can be stored in a file **160a** on the personal repository **101** for later transmission to the central service provider **170.**

In conjunction the collection of real-time biometric and/or medical data from the patient, standardized medical data **150** can be accessed for comparison of the patient's real-time biometric and/or medical data to the expected real-time biometric and/or medical data for the patient to identify any possible health-related abnormalities.

In addition to any of the foregoing, the user can also manually enter personal medical information into the PHR **101a.** Such personal medical information can include but is not limited to, blood type, medications, allergies, upcoming medical appointments, emergency doctors contact information, authorized physicians, organ donor information, advance directives. or any other relevant information.

Specific embodiments of the PHIE system **100** are disclosed by combination of all or a portion of the descriptions of each element of the system.

### Method Related to Personal Repository

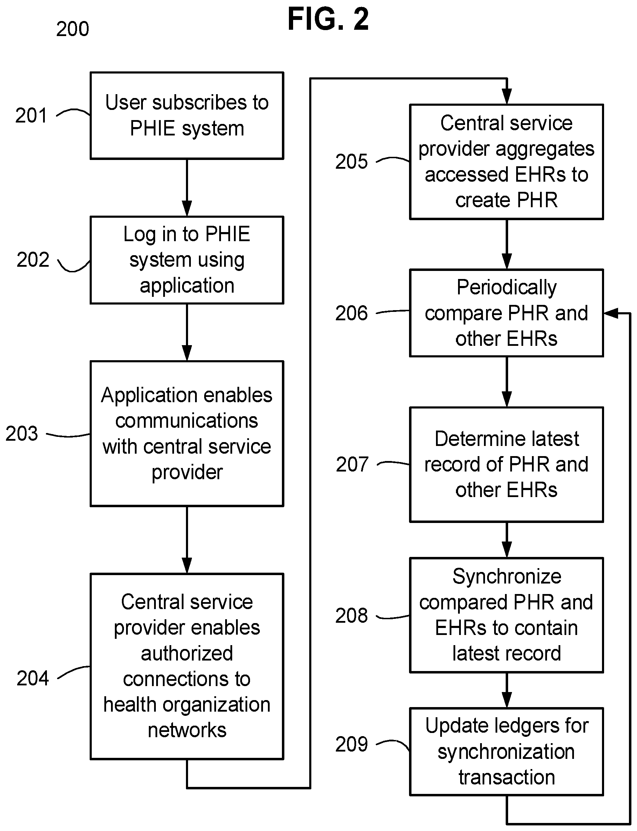

In an embodiment shown in **FIG. 2****,** a method **200** for obtaining a current PHR or personal EHR **101a** using the PHIE system **100** is described. A user, or patient, subscribes **201** to the PHIE system **100** through a website associated with the PHIE system or an application associated with the PHIE system. The user subscribes **201** to the to the PHIE system **100** by entering personal data, including but not limited to name, address, gender, blood type, phone number(s), medical insurance information, emergency contact(s), medication(s), allergies, and/or EHR provider(s). In some embodiments, an application installed on a mobile personal repository is preferred.

A user logs **202** into the PHIE system either through a website associated with the PHIE or an application associated with the PHIE. When the user logs **202** into the PHIE, the website associated with the PHIE or an application associated with the PHIE enables **203** a communication link **101b** with a central service provider **170.** In some embodiments, when a user logs into the PHIE system, the user can elect to maintain the communication link **101b.** In other embodiments, the communications link **101b** is maintained automatically.

After establishing communication link **101b** with the central service provider **170,** the central service provider, through one or more APIs hosted by the central service provider, enables **204** one or more communication links **110b, 120b, 130b,** and/or **140b** with authorized health organization networks **110, 120, 130,** and/or **140.** Health organization networks are authorized when the user adds such networks during the subscription process. Authorized health organization networks **110, 120, 130,** and/or **140** maintain copies of user's EHR **110a, 120a, 130a,** and/or **140a.** The central service provider **170** aggregates **205** information in the EHRs **110a, 120a, 130a,** and/or **140a** to create **205** a PHR **101a** maintained on the user's personal repository **101.**

Once activated and a PHR **101a** exists on personal repository **101,** the PHIE system **100,** periodically and automatically, through APIs hosted on the central service provider **170,** compares **206** PHR **101a** to one or more of copies of user's EHR **110a, 120a, 130a,** and/or **140a.** APIs hosted on the central service provider **170** prompt such comparisons automatically at predetermined time intervals. In some embodiments, the time interval can be once every two weeks, once every week, once every 3 days, once every 2 days, once every 24 hours, or once every 12 hours. Any other time interval can conveniently be programmed in order to achieve the desired functionality of PHIE system **100.**

Preferably, APIs hosted on the central service provider compares the PHR **101a** to all copies of user's EHR **110a, 120a, 130a,** and/or **140a** available at all health organization networks **110, 120, 130,** and/or **140** authorized by the user. Comparison between PHR and other EHRs can be done sequentially or simultaneously. When the PHR **101a** and one or more other EHRs **110a, 120a, 130a,** and/or **140a** are compared, APIs hosted on the central service provider determines **207** which record is later and synchronize **208** the records updating older records such that the PHR **101a** and one or more other EHRs **110a, 120a, 130a,** and/or **140a** all contain the latest information. Ledgers associated with the PHR **101a** and one or more other EHRs **110a, 120a, 130a,** and/or **140a** are then updated **209** to reflect the synchronization transaction.

At times, multiple records may become out of sync, either due to a downtime for part of the system or an update of different records in less time than the time period for the automated synchronization of EHRs. For instance, at a particular time of comparison, PHR **101a** contains A, EHR **110a** contains A+B, and EHR **120a** contains A+C. In this case, EHR **110a** and **120a** are both later records than PHR **101a.** One or more APIs hosted on the central service provider 170 are designed to identify these situations and review the ledgers to identify missing transactions for certain records in order to produce a new record containing A+B+C. Synchronization in this case results in PHR **101a,** EHR **110a,** and EHR **120a** all containing A+B+C. Additionally, data are consolidated and organized into a directory such as, but not limited to, medical history, immunizations, family history, medications, laboratory results, x-rays, and scans, with sources of data identified.

Optionally, the user can also add personal information such as, but not limited to, allergies, medications, organ donor preferences, physical activities, and/or medical appointments, to the PHR **101a** via the PHIE application on the user's personal repository **101.**

### Method Related to Medical Services

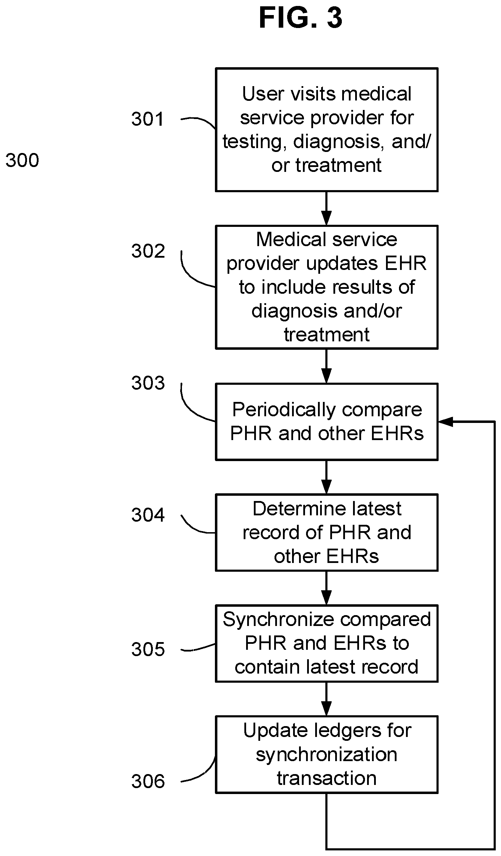

In an embodiment shown in **FIG. 3****,** a method **300** for obtaining a current EHR using the PHIE system **100** is described. A patient can **301** obtain testing, diagnosis, and/or treatment from an medical services provider **111b,** which subscribes to a health organization network **110** that maintains an EHR **110a** for a patient subscribing to the system. After the medical services provider **110** has completed providing services to the patient, the medical services provider **110** documents the testing, diagnosis, and/or treatment in an appointment record. The medical services provider **110** then adds the appointment record to the patient EHR **110a** to create **302** an updated EHR. In some cases, testing, diagnosis, and/or treatment are recorded directly to the EHR to create an updated EHR. Medical services can include ambulatory medical services, routine medical services, or telemedicine services.

The PHIE system **100,** periodically and automatically, through APIs hosted on the central service provider **170,** compares **303** PHR **101a** to one or more of copies of user's EHR **110a, 120a, 130a,** and/or **140a** and determine **304** the latest EHR version. APIs hosted on the central service provider **170** prompt such comparisons automatically at predetermined time intervals. In some embodiments, the time interval can be once every two weeks, once every week, once every 3 days, once every 2 days, once every 24 hours, or once every 12 hours. Any other time interval can conveniently be programmed in order to achieve the desired functionality of PHIE system **100.**

In some embodiments, updating of the patient EHR automatically establishes a communication link **110b** between the health organization network **110** and a central service provider **170.** An API hosted on the central service provider **170** provides a private subnetwork between the health organization network **110** and at least a patient repository device **101** by establishing a communication link **101b** between the central service provider **170** and the patient repository device **101.** One or more APIs hosted on the central service provider **170** synchronize **305** the updated EHR and PHR, such that the PHR contains the same information as the updated EHR, and then update **306** ledgers associated with the EHR and the PHR to record the synchronization transaction.

The central service provider **170** can host one or more APIs that provide for inclusion of at least one additional health organization network **120,** which maintains a patient EHR **120a.** through a communication link **120b.** An API hosted on the central service provider **170** provides a private subnetwork between the health organization network **110,** the patient repository **101,** and the at least one additional health organization network **120** by enabling communication link **120b** between the central service provider **170** and at least one additional health organization network **120.** One or more APIs hosted on the central service provider **170** synchronize the updated EHR **110a ,** PHR **101,** and the EHR **120a,** such that the PHR and EHR **120a** contain the same information as the updated EHR, and then update ledgers associated with the EHR 110a, the PHR **101,** and EHR **120a** to record the synchronization transaction.

A health organization network **110** subscribing to the PHIE system **100,** possesses a copy of the patient's EHR **110a.** The patient's EHR **110a** will match the PHR **101a** on the patient's personal repository **110** at the beginning of the visit to a provider of medical services subscribing to health organization network **110.** After provision of medical services **110** to the patient, the service provider will document all testing, diagnosis, and/or treatment provided during the visit and create an appointment record, which is later added to the patient EHR **110a,** or the patient EHR **110a** is updated directly to include such testing, diagnosis, and/or treatment. An application programming interface hosted by the central service provider **170** automatically and periodically checks all service providers subscribing to the PHIE system **100.** When the change to patient EHR **110a** is detected, the application programming interface will determine the latest EHR version and prompt connections to all other service providers subscribing to the PHIE system **100** through their related communication links to synchronize all other copies of the patient EHR, including the EHR **101a** on patient's personal repository **101,** to match the updated version of patient electronic record **110a** retained by the provider of medical services **110.**

In preferred embodiments, the telemedicine platform not only allows the patient and medical services provider the capability of viewing each other during consultations but also allows bidirectional exchange of files, including, but not limited to, x-rays, ultrasound and/or MRI imaging, uploading of biometric readings from wearable biometric patient monitors, and a private messaging capability offline and online. The transcripts of consultations are automatically pushed to the electronic health record system and to the user's personal repository that becomes part of the user's PHR.

### Method Related to Emergency Services

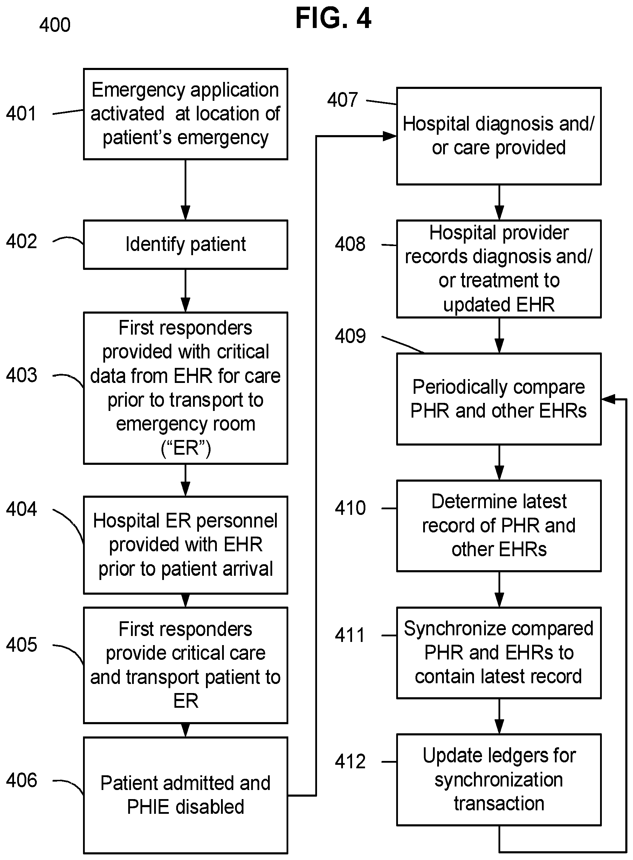

In an embodiment shown in **FIG. 4****,** a method **400** for obtaining a current EHR using the PHIE system **100** is described. When a patient requires emergency medical services, an emergency medical services application on the patient's personal repository **101** can be activated **401** by or on behalf of the patient to facilitate delivery of emergency services to patient. The emergency services can be emergency rooms (at a hospital or other equivalent type facilities) **141, 142** and/or first responders **143.** Efficacy of the emergency health information exchange network (EHIE) **140** improves as the membership of hospitals and first responders to the EHIE increases.

The emergency services application automatically establishes a communication link **101c** between the patient repository device **101** and an emergency health information exchange network (EHIE) **140** that maintains an EHR **140a** for the patient subscribing to the service. If the patient is unconscious, the emergency services application can be activated **401** on the patient repository device **101** by a first responder without the need for an access code or exposing the patient's personal information residing on the patient repository device **101.** If the patient personal repository **101** is unavailable or damaged, the emergency services application can be activated **401** on a repository device of the first responders through communication link **143b.** In some embodiments, first responders' access to limited and possibly life-saving EHR information might be authorized by city, state, and/or federal authorities as being in the public interest. Identification of an unconscious patient can be further added by an identification bracelet, photo identification, pendant, wallet card, or other similar means to indicate emergency services providers **142, 143** participating in the EHIE system **140** in which a patient is a participant.

A patient must be identified **402.** A conscious patient can self-identify. If the patient in unconscious or otherwise unable to self-identify, the EHIE **140** can comprise a communication link to an identification repository. For instance, the identification repository can comprise a master patient index and/or driver registry. The link can be configured to enable the transmissions of user identifying information such as, but not limited to, a photograph of victim, fingerprint, biometric data, and/or other data uniquely identifying an individual. Once identified, all or a portion of the user's EHR, as pre-authorized by the user, can be obtained through communication link **101c** to patient personal repository, through communication link **143b** to first responders **143,** and through communication link **142b** to emergency room (ER) **142** (assuming ER **142** is the only ER involved in this example).

Once the emergency services providers are in contact with the EHIE and the patient has been identified, one or more APIs, hosted on the central service provider **170,** notifies one or more emergency contacts by email and/or short message system, provides **403** critical patient information to first responders and/or provides a private subnetwork between the patient repository device **101** and the hospital emergency room by establishing communication between the central service provider **170** and the emergency room **142** through a communication link **142b** to provide **404** access to EHR **140a.** First responders provide **405** critical care and transport patient to emergency room **142.** The subnetwork is disconnected **406** upon admission of the patient to the hospital. Hospital personnel provide **407** diagnosis and/or care to patient. Upon completion of emergency services, including related hospital stay, and discharge of patient from the hospital **142,** updating the patient EHR **140a** is controlled by the emergency services provider **142** to create **408** an updated EHR **140a** including all emergency services provided.

The PHIE system **100,** periodically and automatically, through APIs hosted on the central service provider **170,** compares **409** PHR **101a** to one or more of copies of user's EHR **110a, 120a, 130a,** and/or **140a** and determine **410** the latest EHR version. APIs hosted on the central service provider **170** prompt such comparisons automatically at predetermined time intervals. In some embodiments, the time interval can be once every two weeks, once every week, once every 3 days, once every 2 days, once every 24 hours, or once every 12 hours. Any other time interval can conveniently be programmed in order to achieve the desired functionality of PHIE system **100.**

In some embodiments, updating of the patient EHR automatically establishes a communication link **140b** between the health organization network **140** and a central service provider **170.** An API hosted on the central service provider **170** provides a private subnetwork between the health organization network **140** and at least a patient repository device **101** by establishing a communication link **101b** between the central service provider **170** and the patient repository device **101.** One or more APIs hosted on the central service provider **411** synchronize the updated EHR and PHR, such that the PHR contains the same information as the updated EHR, and then update **412** ledgers associated with the EHR and the PHR to record the synchronization transaction.

The central service provider **170** can host one or more APIs that provide for inclusion of at least one additional health organization network **120,** which maintains a patient EHR **120a.** through a communication link **120b.** An API hosted on the central service provider **170** provides a private subnetwork between the health organization network **140,** the patient repository **101,** and the at least one additional health organization network **120** by enabling communication link **120b** between the central service provider **170** and at least one additional health organization network **120.** One or more APIs hosted on the central service provider **411** synchronize the updated EHR **140a,** PHR **101,** and the EHR **120a,** such that the PHR and EHR **120a** contain the same information as the updated EHR, and then update ledgers associated with the EHR **140a,** the PHR **101,** and EHR **120a** to record the synchronization transaction.

A health organization network **140** subscribing to the PHIE system **100,** possesses a copy of the patient's EHR **140a.** The patient's EHR **140a** will match the PHR **101a** on the patient's personal repository **140** at the beginning of the visit to a provider of medical services subscribing to health organization network **140.** After provision of medical services **140** to the patient, the service provider will document all testing, diagnosis, and/or treatment provided during the visit and create an appointment record, which is later added to the patient EHR **140a,** or the patient EHR **140a** is updated directly to include such testing, diagnosis, and/or treatment. An application programming interface hosted by the central service provider **170** automatically and periodically checks all service providers subscribing to the PHIE system **100.** When the change to patient EHR **140a** is detected, the application programming interface will prompt connections to all other service providers subscribing to the PHIE system **100** through their related communication links to synchronize all other copies of the patient EHR, including the EHR **101a** on patient's personal repository **101,** to match the updated version of patient electronic record **140a** retained by the EHIE **140.**

### Method Related to Patient Monitors

In another embodiment, one or more patient monitors **160** are used to collect **501** real-time biometric and medical data from a patient. The one or more patient monitors **160** are connected **502** directly **160b** or indirectly **160c-101b** to a central service provider **170.** The real-time biometric and medical data from a patient is transmitted **503** to the central service provider **170** via the enabled connection. One or more APIs hosted on the central service provider **170** perform **504** statistical analysis of patient real-time biometric and medical data and format the results to produce processed patient data. In some embodiments, the processed patient data is added **507** directly to the PHR **101.**

In other embodiments. patient's PHR **101a,** an application programming interface, hosted on the central service provider **170** provides a connection **150b** between the central service provider and a repository of standardized health data **150.** Benchmark data is downloaded **505** from the repository of standardized health data **150** to the central service provider **170.** One or more APIs on the central service provider **170** compares **506** processed patient data to benchmark data to identify possible health abnormalities in the patient and results are added **507** to the PHR **101a.**

It is not intended that the scope of the claims appended hereto be limited to the examples and descriptions set forth herein but rather that the claims be construed as encompassing all the features of patentable novelty which reside herein.

In the preceding description, the present disclosure is described with reference to specifically exemplary embodiments thereof. The specification and drawings are, accordingly, to be regarded as illustrative and not as restrictive. It is understood that the present disclosure is capable of using various other combinations and embodiments and is capable of any changes or modifications within the scope of the inventive concept as expressed herein.

## Claims

1. A system enabling transmission of a medical history, comprising: a plurality of health organization networks, at least one of the health organization networks comprising a repository holding a medical history; a plurality of ledgers encoding changes to the medical history; a central service provider connected to each of the plurality of health organization networks via a communication link associated with each health organization network; and a server enhancement hosted by the central service provider, the server enhancement configured to: provide a private subnetwork between at least a portion of the health organization networks through all or a portion of the communication links; and direct maintenance of at least one ledger by the health organization networks to reflect changes to the medical history, **characterized in that** the system comprises a network of patient monitors configured to communicate with one or more of the health organization networks via the central service provider through the communication link.

2. The system of claim 1, wherein the health organization networks comprise at least one of clinics, hospitals, and offices and/or wherein the communication link comprises one or more of telephone lines, satellite links, and radio transmissions.

3. The system of claim 1, further comprising a personal repository connection with one or more of the health organization networks via the communication link related to the personal repository and the communication links related to the one or more health organization networks connected through the central service provider.

4. The system of claim 1, wherein the server enhancement is further configured to transiently store at least one of information identifying a patient, information identifying a document, and a transmitted medical history to facilitate the update of the at least one ledger.

5. A method for obtaining a current electronic health record (EHR), the method comprising: providing a personal health information exchange (PHIE) to a patient, the PHIE comprising: a personal repository containing a PHR of the patient; one or more health organization networks, wherein each network maintains an EHR of the patient and comprises connections to one or more medical services providers selected from one or more of each ambulatory services, emergency services, telemedicine services, routine medical services and/or any combination thereof; a server enhancement, hosted on a central service provider: providing a subnetwork between the personal repository and the one or more health organization networks through communication links associated with the personal repository and the one or more health organization networks; providing automatic review of the subnetwork at a specific time interval to identify a change to any EHR; providing synchronization of the EHRs in response to new data being added to a single EHR; and providing for updates to ledgers associated with each EHR to record changes to each EHR; the patient causing an update to one of the EHRs by obtaining medical services form a medical services provider; and the patient obtaining and updated PHR after a specific time interval.

6. The method of claim 5 comprising: an application on a personal repository automatically enabling a communication link with a central service provider, wherein the personal repository contains a first EHR of a patient; and a server enhancement, hosted on the central service provider: providing a private subnetwork between the personal repository and at least a first health organization network by enabling a communication link between the central service provider and the first health organization network, wherein the first health organization network contains a second EHR of a patient; comparing the first EHR and the second EHR to determine which EHR has the more current data; synchronizing the first EHR and the second EHR, such that both EHRs contain the more current data; and updating a ledger associated with each EHR to record the transaction of synchronizing the first EHR and the second EHR.

7. The method of claim 5, wherein the server enhancement, hosted on the central service provider, further: provides a private subnetwork between the patient repository device, the first health organization network, and at least a second health organization network by enabling a communication link between the central service provider and the second health organization network, wherein the second health organization network contains a third EHR of a patient; comparing the first EHR, the second EHR, and the third EHR to determine which EHR has the most current data; synchronizing the first EHR, the second EHR, and the third EHR, such that all three EHRs contain the most current data; and updating a ledger associated with each EHR to record the transaction of synchronizing all three EHRs.

8. The method of claim 5, wherein the server enhancement, hosted on the central service provider, further: provides a private subnetwork between the patient repository device, the first health organization network, the second health organization network, and at least a third health organization network by enabling a communication link between the central service provider and the third health organization network, wherein the third health organization network contains a fourth EHR of a patient; comparing the first EHR, the second EHR, the third EHR, and the fourth EHR to determine which EHR has the most current data; synchronizing the first EHR, the second EHR, the third EHR, and the fourth EHR, such that all four EHRs contain the most current data; and updating a ledger associated with each EHR to record the transaction of synchronizing all four EHRs.

9. The method of claim 5 comprising: a user creating an updated first EHR on a personal repository by adding information to the first EHR through an application on the personal repository; the application on the personal repository automatically establishing a communication link with a central service provider; and a server enhancement, hosted on the central service provider: providing a private subnetwork between the personal repository and at least a first health organization network by enabling a communication link between the central service provider and the first health organization network, wherein the first health organization network contains a second EHR of a patient; synchronizing the first EHR and the second EHR, such that both EHRs contain the information of the updated first EHR; and updating a ledger associated with each EHR to record the transaction of synchronizing the first EHR and the second EHR.

10. The method of claim 5 comprising: a patient receiving testing, diagnosis, and/or treatment from a medical services provider, wherein the medical services provider subscribes to a first health organization network, which maintains a first EHR for the patient; the medical services provider documenting the testing, diagnosis, and/or treatment in the patient EHR to create an updated first EHR; a server enhancement, hosted on the central service provider: providing a private subnetwork between the first health organization network and a patient repository device by enabling a communication link between the central service provider and the patient repository device, wherein a PHR resides on the patient repository device; comparing the updated first EHR and the PHR to identify that the updated first EHR has more current data; synchronizing the updated first EHR and the PHR, such that both EHRs contain the more current data; and updating a ledger associated with each EHR to record the transaction of synchronizing the first EHR and the PHR.

11. The method of claim 10, wherein the server enhancement, hosted on the central service provider, further: provides a private subnetwork between the first health organization network, the patient repository device, and at least a second health organization network by enabling a communication between the central service provider and the second health organization network, wherein the second health organization network maintains a second EHR; comparing the updated first EHR, the PHR, and the second EHR to identify that the updated first EHR has more current data; synchronizing the updated first EHR, PHR, and second EHR, such that the PHR and the second EHR each contain the information of the updated first EHR; and updating a ledger associated with each EHR and the PHR to record the transaction of synchronizing the EHR and the PHR.

12. The method of claim 10, wherein the medical services provider is an ambulatory medical services provider, a telemedicine services provider, or a routine medical services provider.

13. The method of claim 5 comprising: providing a personal health information exchange (PHIE) system comprising: an emergency medical services application, an emergency health information network (EHIE), a patient EHR stored on the EHIE, and a personal repository; activating the emergency services application; identifying patient; and a server enhancement, hosted on a central service provider: providing first responders with critical data from EHR by enabling a private communication link between the first responders and the EHIE; and providing emergency room personnel access to EHR by enabling a private communication link between the emergency room personnel and the EHIE; first responders providing critical care and transporting patient to emergency room; a second server enhancement, hosted on the central service provider: disconnecting the PHIE when patient is admitted to emergency room and/or hospital; emergency room and/or hospital personnel providing diagnosis and/or care to patient; emergency room and/or hospital personnel recording all emergency medical services provided to patient to create updated EHR; and a third server enhancement, hosted on the central service provider: providing a private subnetwork between the EHIE and a patient repository device by enabling a communication link between the central service provider and the patient repository device, wherein a PHR resides on the patient repository device; comparing the updated EHR and the PHR to identify that the updated EHR has more current data; synchronizing the updated EHR and the PHR, such that both EHRs contain the more current data; and updating a ledger associated with the EHR and PHR to record the transaction of synchronizing the EHR and the PHR.

14. The method of claim 13, wherein the first server enhancement, hosted on the central service provider, further provides for: notifying one or more emergency contacts by email and/or short message system.

15. The method of claim 5 comprising: providing a personal health information exchange (PHIE) system comprising a personal health record stored on a personal repository and at least on patient monitor; a patient monitor collecting real-time biometric and/or medical data from a patient; delivering real-time biometric and/or medical data from a patient to a central service provider; and a server enhancement, hosted on the central service provider: providing for processing, statistical analysis, and formatting of patient real-time biometric and medical data to produce processed patient data; and providing for addition of the processed patient data to the PHR, wherein the server enhancement, hosted on the central service provider, further provides for: providing a private subnetwork between the patient repository device and a repository of standardized health data by enabling a communication link between the central service provider and a repository of standardized health data; extracting benchmark data from the repository of standardized health data; comparing the processed patient data to the benchmark data to identify possible health-related abnormalities with the patient to produce benchmark report; and providing for addition of the benchmark report to the PHR.

## Patentansprüche

1. System, das die Übermittlung einer Krankengeschichte ermöglicht, umfassend: eine Vielzahl von Gesundheitsorganisationsnetzwerken, wobei mindestens eines der Gesundheitsorganisationsnetzwerke einen Speicher umfasst, der eine Krankengeschichte enthält; eine Vielzahl von Hauptbüchern, die Änderungen an der Krankengeschichte kodieren; einen zentralen Dienstleister, der mit jedem der Vielzahl von Gesundheitsorganisationsnetzwerken über eine Kommunikationsverbindung verbunden ist, die mit jedem Gesundheitsorganisationsnetzwerk verknüpft ist; und eine Servererweiterung, die von dem zentralen Dienstleister gehostet wird, wobei die Servererweiterung konfiguriert ist, um: ein privates Teilnetzwerk zwischen mindestens einem Teil der Gesundheitsorganisationsnetzwerke über alle oder einen Teil der Kommunikationsverbindungen bereitzustellen; und eine direkte Verwaltung mindestens eines Hauptbuchs durch die Gesundheitsorganisationsnetzwerke, um Änderungen der Krankengeschichte widerzuspiegeln, **dadurch gekennzeichnet, dass** das System ein Netzwerk von Patientenmonitoren umfasst, das so konfiguriert ist, dass es über den zentralen Dienstleister über die Kommunikationsverbindung mit einem oder mehreren Gesundheitsorganisationsnetzwerken kommuniziert.

2. System gemäß Anspruch 1, wobei die Gesundheitsorganisationsnetzwerke mindestens eines von Kliniken, Krankenhäuser und Praxen umfassen und/oder wobei die Kommunikationsverbindung eine oder mehrere Telefonleitungen, Satellitenverbindungen und Funkübertragungen umfasst.

3. System gemäß Anspruch 1, ferner umfassend eine persönliche Speicherverbindung mit einem oder mehreren Gesundheitsorganisationsnetzwerken über die Kommunikationsverbindung, die sich auf den persönlichen Speicher bezieht, und die Kommunikationsverbindungen, die sich auf das eine oder die mehreren Gesundheitsorganisationsnetzwerke beziehen, die über den zentralen Dienstleister verbunden sind.

4. System gemäß Anspruch 1, wobei die Servererweiterung ferner so konfiguriert ist, dass sie vorübergehend mindestes eine Information, die einen Patienten oder ein Dokument identifiziert, und eine übermittelte Krankengeschichte speichert, um die Aktualisierung des mindestens einen Hauptbuchs zu erleichtern.

5. Verfahren zum Abrufen einer aktuellen elektronischen Gesundheitsakte (EHR), wobei das Verfahren umfasst: Bereitstellen eines persönlichen Austausch an Gesundheitsinformationen (PHIE) für einen Patienten, wobei der PHIE umfasst: einen persönlichen Speicher, der eine persönliche Gesundheitsakte (PHR) des Patienten enthält; ein oder mehrere Gesundheitsorganisationsnetzwerke, wobei jedes Netzwerk eine EHR des Patienten verwaltet und Verbindungen zu einem oder mehreren medizinischen Dienstleistern umfasst, die jeweils aus einem oder mehreren der folgenden Dienste ausgewählt sind: ambulante Dienste, Notfalldienste, telemedizinische Dienste, medizinische Routinedienste und/oder eine beliebige Kombination davon; eine Servererweiterung, die bei einem zentralen Dienstleister gehostet wird: Bereitstellen eines Teilnetzwerks zwischen dem persönlichen Speicher und dem einem oder den mehreren Gesundheitsorganisationsnetzwerken über Kommunikationsverbindungen, die mit dem persönlichen Speicher und dem einem oder den mehreren Gesundheitsorganisationsnetzwerken verknüpft sind; Bereitstellen einer automatischen Überprüfung des Teilnetzwerks in einem bestimmten Zeitintervall, um eine Änderung an einer EHR zu erkennen; Bereitstellen einer Synchronisierung der EHRs als Reaktion auf neue Daten, die zu einer einzelnen EHR hinzugefügt werden; und Bereitstellen von Aktualisierungen der Hauptbücher, die mit jeder EHR verknüpft sind, um Änderungen an jeder EHR aufzuzeichnen; wobei der Patient eine Aktualisierung einer der EHRs auslöst, indem er medizinische Dienste von einem medizinischen Dienstleister in Anspruch nimmt; und der Patient eine aktualisierte PHR nach einem bestimmten Zeitintervall erhält.

6. Verfahren gemäß Anspruch 5, umfassend: eine Anwendung auf einem persönlichen Speicher, der automatisch eine Kommunikationsverbindung mit einem zentralen Dienstleister ermöglicht, wobei der persönliche Speicher eine erste EHR eines Patienten enthält; und eine Servererweiterung, die bei dem zentralen Dienstleister gehostet wird: Bereitstellen eines privaten Teilnetzwerks zwischen dem persönlichen Speicher und dem mindestens einem ersten Gesundheitsorganisationsnetzwerk, indem eine Kommunikationsverbindung zwischen dem zentralen Dienstleister und dem ersten Gesundheitsorganisationsnetzwerk ermöglicht wird, wobei das erste Gesundheitsorganisationsnetzwerk eine zweite EHR eines Patienten enthält; Vergleichen der ersten EHR und der zweiten EHR, um herauszufinden, welche EHR die aktuelleren Daten enthält; Synchronisieren der ersten EHR und der zweiten EHR, sodass beide EHRs die aktuelleren Daten enthalten; und Aktualisieren eines mit jeder EHR verknüpften Hauptbuchs, um den Synchronisierungsvorgang der ersten EHR und der zweiten EHR aufzuzeichnen.

7. Verfahren gemäß Anspruch 5, wobei die Servererweiterung, die bei dem zentralen Dienstleister gehostet wird, ferner umfasst: Bereitstellen eines privaten Teilnetzwerks zwischen dem Patientenspeichergerät, dem ersten Gesundheitsorganisationsnetzwerk und dem mindestens zweitem Gesundheitsorganisationsnetzwerk, indem eine Kommunikationsverbindung zwischen dem zentralen Dienstleister und dem zweiten Gesundheitsorganisationsnetzwerk ermöglicht wird, wobei das zweite Gesundheitsorganisationsnetzwerk eine dritte EHR eines Patienten enthält; Vergleichen der ersten EHR, der zweiten EHR und der dritten EHR, um herauszufinden, welche EHR die aktuellsten Daten enthält; Synchronisieren der ersten EHR, der zweiten EHR und der dritten EHR, sodass alle drei EHRs die aktuellsten Daten enthalten; und Aktualisieren eines mit jeder EHR verknüpften Hauptbuchs, um den Synchronisierungsvorgang aller drei EHRs aufzuzeichnen.

8. Verfahren gemäß Anspruch 5, wobei die Servererweiterung, die bei dem zentralen Dienstleister gehostet wird, ferner umfasst: Bereitstellen eines privaten Teilnetzwerks zwischen dem Patientenspeichergerät, dem ersten Gesundheitsorganisationsnetzwerk, dem zweitem Gesundheitsorganisationsnetzwerk und dem mindestens dritten Gesundheitsorganisationsnetzwerk, indem eine Kommunikationsverbindung zwischen dem zentralen Dienstleister und dem dritten Gesundheitsorganisationsnetzwerk ermöglicht wird, wobei das dritte Gesundheitsorganisationsnetzwerk eine vierte EHR eines Patienten enthält; Vergleichen der ersten EHR, der zweiten EHR, der dritten EHR und der vierten EHR, um herauszufinden, welche EHR die aktuellsten Daten enthält; Synchronisieren der ersten EHR, der zweiten EHR, der dritten EHR und der vierten EHR, sodass alle vier EHRs die aktuellsten Daten enthalten; und Aktualisieren eines mit jeder EHR verknüpften Hauptbuchs, um den Synchronisierungsvorgang aller vier EHRs aufzuzeichnen.

9. Verfahren gemäß Anspruch 5, umfassend: ein Benutzer, der eine aktualisierte erste EHR auf einem persönlichen Speicher erstellt, indem er über eine Anwendung auf dem persönlichen Speicher Informationen zur ersten EHR hinzufügt; wobei die Anwendung auf dem persönlichen Speicher automatisch eine Kommunikationsverbindung mit einem zentralen Dienstleister herstellt; und eine Servererweiterung, die bei dem zentralen Dienstleister gehostet wird: Bereitstellen eines privaten Teilnetzwerks zwischen dem persönlichen Speicher und dem mindestens ersten Gesundheitsorganisationsnetzwerk, indem eine Kommunikationsverbindung zwischen dem zentralen Dienstleister und dem ersten Gesundheitsorganisationsnetzwerk ermöglicht wird, wobei das erste Gesundheitsorganisationsnetzwerk eine zweite EHR eines Patienten enthält; Synchronisieren der ersten EHR und der zweiten EHR, sodass beide EHRs die Informationen der aktualisierten ersten EHR enthalten; und Aktualisieren eines mit jeder EHR verknüpften Hauptbuchs, um den Synchronisierungsvorgang der ersten EHR und der zweiten EHR aufzuzeichnen.

10. Verfahren gemäß Anspruch 5, umfassend: ein Patient, der Untersuchungen, Diagnosen und/oder Behandlungen von einem medizinischen Dienstleister erhält, wobei der medizinische Dienstleister sich bei einem ersten Gesundheitsorganisationsnetzwerk registriert, das eine erste EHR für den Patienten verwaltet; der medizinische Dienstleister dokumentiert die Untersuchungen, Diagnosen und/oder Behandlungen in der EHR des Patienten, um eine aktualisierte erste EHR zu erzeugen; eine Servererweiterung, die bei dem zentralen Dienstleister gehostet wird: Bereitstellen eines privaten Teilnetzwerks zwischen dem ersten Gesundheitsorganisationsnetzwerk und einem Patientenspeichergerät, indem eine Kommunikationsverbindung zwischen dem zentralen Dienstleister und dem Patientenspeichergerät ermöglicht wird, wobei sich eine PHR auf dem Patientenspeichergerät befindet; Vergleichen der aktualisierten ersten EHR und der PHR, um herauszufinden, ob die aktualisierte erste EHR aktuellere Daten enthält; Synchronisieren der aktualisierten ersten EHR und der PHR, sodass beide EHRs die aktuelleren Daten enthalten; und Aktualisieren eines mit jeder EHR verknüpften Hauptbuchs, um den Synchronisierungsvorgang der ersten EHR und der PHR aufzuzeichnen.

11. Verfahren gemäß Anspruch 10, wobei die Servererweiterung, die bei dem zentralen Dienstleister gehostet wird, ferner umfasst: Bereitstellen eines privaten Teilnetzwerkes zwischen dem ersten Gesundheitsorganisationsnetzwerk, dem Patientenspeichergerät und dem mindestens zweitem Gesundheitsorganisationsnetzwerk, indem eine Kommunikationsverbindung zwischen dem zentralen Dienstleister und dem zweiten Gesundheitsorganisationsnetzwerk ermöglicht wird, wobei das zweite Gesundheitsorganisationsnetzwerk eine zweite EHR verwaltet; Vergleichen der aktualisierten ersten EHR, der PHR und der zweiten EHR, um herauszufinden, ob die aktualisierte erste EHR die aktuelleren Daten enthält; Synchronisieren der aktualisierten ersten EHR, der PHR und der zweiten EHR, sodass die PHR und die zweite EHR jeweils die Informationen der aktualisierten ersten EHR enthalten; und Aktualisieren eines Hauptbuchs, das mit jeder EHR und der PHR verknüpft ist, um den Synchronisierungsvorgang der EHR und der PHR aufzuzeichnen.

12. Verfahren gemäß Anspruch 10, wobei der medizinische Dienstleister ein ambulanter medizinischer Dienstleister, ein telemedizinischer Dienstleister oder ein medizinischer Routinedienstleister ist.

13. Verfahren gemäß Anspruch 5, umfassend: Bereitstellen eines Systems zum Austausch persönlicher Gesundheitsinformationen (PHIE), umfassend: eine Anwendung für medizinische Notfalldienste, ein Netzwerk für medizinische Notfallinformationen (EHIE), eine auf dem EHIE gespeicherte EHR eines Patienten und ein persönlicher Speicher; Aktivieren der Anwendung für Notfalldienste; Identifizieren des Patienten; und eine Servererweiterung, die bei einem zentralen Dienstleister gehostet wird: Versorgen von Ersthelfern mit wichtigen Daten aus der EHR, indem eine private Kommunikationsverbindung zwischen den Ersthelfern und dem EHIE ermöglicht wird; und Ermöglichen des Zugriffs des Notaufnahmepersonals auf die EHR, indem eine private Kommunikationsverbindung zwischen dem Notaufnahmepersonal und dem EHIE ermöglicht wird; Ersthelfer, die Erste Hilfe leisten und Patienten in die Notaufnahme bringen; eine zweite Servererweiterung, die bei dem zentralen Dienstleister gehostet wird: Trennen des PHIE, wenn der Patient in die Notaufnahme und/oder ins Krankenhaus eingeliefert wird; Notaufnahme- und/oder Krankenhauspersonal, das den Patienten diagnostiziert und/oder pflegt; Notaufnahme- und/oder Krankenhauspersonal, das alle medizinischen Notfalldienste, die dem Patienten erbracht wurden, aufzeichnet, um eine aktualisierte EHR zu erstellen; und eine dritte Servererweiterung, die bei dem zentralen Dienstleister gehostet wird: Bereitstellen eines privaten Teilnetzwerks zwischen dem EHIE und einem Patientenspeichergerät, indem eine Kommunikationsverbindung zwischen dem zentralen Dienstleister und dem Patientenspeichergerät ermöglicht wird, wobei sich eine EHR auf dem Patientenspeichergerät befindet; Vergleichen der aktualisierten EHR und der PHR, um herauszufinden, ob die aktualisierte EHR aktuellere Daten enthält; Synchronisieren der aktualisierten EHR und PHR, sodass beide EHRs die aktuelleren Daten enthalten; und Aktualisieren eines Hauptbuchs, das mit der EHR und der PHR verknüpft ist, um den Synchronisierungsvorgang der EHR und der PHR aufzuzeichnen.

14. Verfahren gemäß Anspruch 13, wobei die erste Servererweiterung, die bei dem zentralen Dienstleister gehostet wird, ferner umfasst: Benachrichtigen eines oder mehrerer Notfallkontakte per E-Mail und/oder SMS.

15. Verfahren gemäß Anspruch 5, umfassend: Bereitstellen eines Systems zum Austausch persönlicher Gesundheitsinformationen (PHIE), umfassend eine persönliche Gesundheitsakte, die auf einem persönlichen Speicher und mindestens einem Patientenmonitor gespeichert ist; einen Patientenmonitor, der biometrische und/oder medizinische Daten eines Patienten in Echtzeit sammelt; Übermittlung biometrischer und/oder medizinischer Daten eines Patienten in Echtzeit an einen zentralen Dienstleister; und eine Servererweiterung, die bei einem zentralen Dienstleister gehostet wird: Bereitstellen der Verarbeitung, statistischen Analyse und Formatierung biometrischer und medizinischer Patientendaten in Echtzeit, um verarbeite Patientendaten zu erstellen; und Hinzufügen von verarbeiteten Patientendaten zur EHR, wobei die Servererweiterung, die bei einem zentralen Dienstleister gehostet wird, ferner umfasst: Bereitstellen eines privaten Teilnetzwerks zwischen dem Patientenspeichergerät und einem Speicher mit standardisierten Gesundheitsinformationen, indem eine Kommunikationsverbindung zwischen dem zentralen Dienstleister und einem Speicher mit standardisierten Gesundheitsinformationen ermöglicht wird; Entnehmen von Eckdaten aus dem Speicher mit standardisierten Gesundheitsinformationen; Vergleichen der verarbeiteten Patientendaten mit den Eckdaten, um mögliche gesundheitsbezogene Anomalien beim Patienten zu ermitteln, um einen Eckdatenbericht zu erstellen; und Hinzufügen des Eckdatenberichts zur PHR.

## Revendications

1. Système permettant la transmission d'un historique médical constitué d'antécédents médicaux, lequel système comprend : une pluralité de réseaux d'organismes de santé, au moins l'un des réseaux d'organismes de santé comprenant un moyen d'archivage qui contient des antécédents médicaux ; une pluralité de registres qui codent des modifications qui sont apportées aux antécédents médicaux ; un fournisseur centralisé de services qui est connecté à chacun de la pluralité de réseaux d'organismes de santé via une liaison de communication qui est associée à chaque réseau d'organismes de santé ; et un moyen de rehaussement de serveur qui est hébergé par le fournisseur centralisé de services, le moyen de rehaussement de serveur étant configuré pour assurer : la constitution d'un sous-réseau privé entre au moins une partie des réseaux d'organismes de santé par l'intermédiaire de la totalité ou d'une partie des liaisons de communication ; et la direction du stockage et de l'actualisation d'au moins un registre par les réseaux d'organismes de santé afin de refléter les modifications qui sont apportées aux antécédents médicaux, **caractérisé en ce que** le système comprend un réseau de moniteurs de patient qui sont configurés pour communiquer avec un ou plusieurs des réseaux d'organismes de santé via le fournisseur centralisé de services par l'intermédiaire de la liaison de communication.

2. Système selon la revendication 1, dans lequel les réseaux d'organismes de santé comprennent au moins un organisme de santé parmi les cliniques, les hôpitaux et les cabinets médicaux ou de soin et/ou dans lequel la liaison de communication comprend un ou plusieurs moyens de liaison de communication parmi les lignes téléphoniques, les liaisons par satellite(s) et les transmissions par radio.

3. Système selon la revendication 1, comprenant en outre une connexion de moyen d'archivage personnel avec un ou plusieurs des réseaux d'organismes de santé via la liaison de communication qui est en relation avec le moyen d'archivage personnel et via les liaisons de communication qui sont en relation avec les un ou plusieurs réseaux d'organismes de santé qui sont connectés par l'intermédiaire du fournisseur centralisé de services.

4. Système selon la revendication 1, dans lequel le moyen de rehaussement de serveur est en outre configuré pour stocker de manière transitoire au moins un ensemble informationnel parmi une information qui identifie un patient, une information qui identifie un document et des antécédents médicaux transmis afin de faciliter la mise à jour de l'au moins un registre.

5. Procédé pour obtenir un enregistrement de santé électronique (EHR) courant, le procédé comprenant : la fourniture à un patient d'un moyen d'échange d'informations personnelles concernant la santé (PHIE), le PHIE comprenant : un moyen d'archivage personnel qui contient un enregistrement personnel concernant la santé, soit un PHR, du patient ; un ou plusieurs réseaux d'organismes de santé, dans lequel chaque réseau stocke et actualise un EHR du patient et comprend des connexions sur un ou plusieurs fournisseurs de services de soins médicaux, lesquels services de soins médicaux sont sélectionnés parmi un ou plusieurs services de soins médicaux que sont les services de soins médicaux ambulatoires, les services de soins médicaux d'urgence, les services de soins médicaux par télémédecine, les services de soins médicaux de routine et/ou une quelconque combinaison de ceux-ci ; un moyen de rehaussement de serveur, qui est hébergé sur un fournisseur centralisé de services et qui assure : la constitution d'un sous-réseau entre le moyen d'archivage personnel et les un ou plusieurs réseaux d'organismes de santé par l'intermédiaire de liaisons de communication qui sont associées au moyen d'archivage personnel et aux un ou plusieurs réseaux d'organismes de santé ; la réalisation d'un examen automatique du sous-réseau selon un intervalle de temps spécifique afin d'identifier une modification qui est apportée à un quelconque EHR ; la réalisation de la synchronisation des EHR en réponse au fait que de nouvelles données sont ajoutées à un unique EHR ; et la réalisation des mises à jour sur les registres qui sont associés à chaque EHR afin d'enregistrer les modifications qui sont apportées à chaque EHR ; le patient effectuant une mise à jour au niveau de l'un des EHR en obtenant des services de soins médicaux de la part d'un fournisseur de services de soins médicaux ; et le patient obtenant un PHR mis à jour après un intervalle de temps spécifique.

6. Procédé selon la revendication 5, comprenant : une application sur un moyen d'archivage personnel qui permet de manière automatique une liaison de communication avec un fournisseur centralisé de services, dans lequel le moyen d'archivage personnel contient un premier EHR d'un patient ; et un moyen de rehaussement de serveur, qui est hébergé sur le fournisseur centralisé de services et qui assure : la constitution d'un sous-réseau privé entre le moyen d'archivage personnel et au moins un premier réseau d'organismes de santé en permettant une liaison de communication entre le fournisseur centralisé de services et le premier réseau d'organismes de santé, dans lequel le premier réseau d'organismes de santé contient un deuxième EHR d'un patient ; la comparaison du premier EHR et du deuxième EHR afin de déterminer le EHR qui dispose des données plus récentes ; la synchronisation du premier EHR et du deuxième EHR de telle sorte que les deux EHR contiennent les données plus récentes ; et la mise à jour d'un registre qui est associé à chaque EHR afin d'enregistrer la transaction de synchronisation du premier EHR et du deuxième EHR.

7. Procédé selon la revendication 5, dans lequel le moyen de rehaussement de serveur, qui est hébergé sur le fournisseur centralisé de services, assure en outre : la constitution d'un sous-réseau privé entre le dispositif de moyen d'archivage de patient, le premier réseau d'organismes de santé et au moins un deuxième réseau d'organismes de santé en permettant une liaison de communication entre le fournisseur centralisé de services et le deuxième réseau d'organismes de santé, dans lequel le deuxième réseau d'organismes de santé contient un troisième EHR d'un patient ; la comparaison du premier EHR, du deuxième EHR et du troisième EHR afin de déterminer le EHR qui dispose des données plus récentes ; la synchronisation du premier EHR, du deuxième EHR et du troisième EHR de telle sorte que les trois EHR contiennent tous les données plus récentes ; et la mise à jour d'un registre qui est associé à chaque EHR afin d'enregistrer la transaction de synchronisation de l'ensemble des trois EHR.

8. Procédé selon la revendication 5, dans lequel le moyen de rehaussement de serveur, qui est hébergé sur le fournisseur centralisé de services, assure en outre : la constitution d'un sous-réseau privé entre le dispositif de moyen d'archivage de patient, le premier réseau d'organismes de santé, le deuxième réseau d'organismes de santé et au moins un troisième réseau d'organismes de santé en permettant une liaison de communication entre le fournisseur centralisé de services et le troisième réseau d'organismes de santé, dans lequel le troisième réseau d'organismes de santé contient un quatrième EHR d'un patient ; la comparaison du premier EHR, du deuxième EHR, du troisième EHR et du quatrième EHR afin de déterminer le EHR qui dispose des données plus récentes ; la synchronisation du premier EHR, du deuxième EHR, du troisième EHR et du quatrième EHR de telle sorte que les quatre EHR contiennent tous les données plus récentes ; et la mise à jour d'un registre qui est associé à chaque EHR afin d'enregistrer la transaction de synchronisation de l'ensemble des quatre EHR.

9. Procédé selon la revendication 5, comprenant : la création par un utilisateur d'un premier EHR mis à jour sur un moyen d'archivage personnel en ajoutant des informations au premier EHR par l'intermédiaire d'une application située sur le moyen d'archivage personnel ; l'application située sur le moyen d'archivage personnel établissant de manière automatique une liaison de communication avec un fournisseur centralisé de services ; et un moyen de rehaussement de serveur, qui est hébergé sur le fournisseur centralisé de services et qui assure : la constitution d'un sous-réseau privé entre le moyen d'archivage personnel et au moins un premier réseau d'organismes de santé en permettant une liaison de communication entre le fournisseur centralisé de services et le premier réseau d'organismes de santé, dans lequel le premier réseau d'organismes de santé contient un deuxième EHR d'un patient ; la synchronisation du premier EHR et du deuxième EHR de telle sorte que les deux EHR contiennent les informations du premier EHR mis à jour ; et la mise à jour d'un registre qui est associé à chaque EHR afin d'enregistrer la transaction de synchronisation du premier EHR et du deuxième EHR.

10. Procédé selon la revendication 5, comprenant : la réception par un patient d'un test, d'un diagnostic et/ou d'un traitement en provenance d'un fournisseur de services de soins médicaux, dans lequel le fournisseur de services de soins médicaux a souscrit un abonnement à un premier réseau d'organismes de santé, lequel assure le stockage et l'actualisation d'un premier EHR pour le patient ; le fournisseur de services de soins médicaux documentant le test, le diagnostic et/ou le traitement dans le EHR du patient afin de créer un premier EHR mis à jour ; un moyen de rehaussement de serveur, qui est hébergé sur le fournisseur centralisé de services et qui assure : la constitution d'un sous-réseau privé entre le premier réseau d'organismes de santé et un dispositif de moyen d'archivage de patient en permettant une liaison de communication entre le fournisseur centralisé de services et le dispositif de moyen d'archivage de patient, dans lequel un PHR est situé sur le dispositif de moyen d'archivage de patient ; la comparaison du premier EHR mis à jour et du PHR afin d'identifier que le premier EHR mis à jour dispose de données plus récentes ; la synchronisation du premier EHR mis à jour et du PHR de telle sorte que les deux EHR contiennent les données plus récentes ; et la mise à jour d'un registre qui est associé à chaque EHR afin d'enregistrer la transaction de synchronisation du premier EHR et du PHR.

11. Procédé selon la revendication 10, dans lequel le moyen de rehaussement de serveur, qui est hébergé sur le fournisseur centralisé de services, assure en outre : la constitution d'un sous-réseau privé entre le premier réseau d'organismes de santé, le dispositif de moyen d'archivage de patient et au moins un deuxième réseau d'organismes de santé en permettant une communication entre le fournisseur centralisé de services et le deuxième réseau d'organismes de santé, dans lequel le deuxième réseau d'organismes de santé assure le stockage et l'actualisation d'un deuxième EHR ; la comparaison du premier EHR mis à jour, du PHR et du deuxième EHR afin d'identifier que le premier EHR mis à jour dispose de données plus récentes ; la synchronisation du premier EHR mis à jour, du PHR et du deuxième EHR de telle sorte que le PHR et le deuxième EHR contiennent chacun les informations du premier EHR mis à jour ; et la mise à jour d'un registre qui est associé à chaque EHR et au PHR afin d'enregistrer la transaction de synchronisation du EHR et du PHR.

12. Procédé selon la revendication 10, dans lequel le fournisseur de services de soins médicaux est un fournisseur de services de soins médicaux ambulatoires, un fournisseur de services de soins médicaux par télémédecine ou un fournisseur de services de soins médicaux de routine.

13. Procédé selon la revendication 5, comprenant : la fourniture d'un système d'échange d'informations personnelles concernant la santé (PHIE) comprenant : une application de services de soins médicaux d'urgence, un réseau d'informations de santé d'urgence (EHIE), un EHR de patient qui est stocké sur le EHIE et un moyen d'archivage personnel ; l'activation de l'application de services de soins médicaux d'urgence ; l'identification d'un patient ; et un moyen de rehaussement de serveur, qui est hébergé sur un fournisseur centralisé de services et qui assure : la fourniture à des premiers intervenants de données indispensables qui proviennent d'un EHR en permettant une liaison de communication privée entre les premiers intervenants et le EHIE ; et la fourniture, au personnel de la salle des urgences, d'un accès au EHR en permettant une liaison de communication privée entre le personnel de la salle des urgences et le EHIE ; les premiers intervenants prodiguant les soins indispensables et transportant le patient jusqu'à la salle des urgences ; un deuxième moyen de rehaussement de serveur, qui est hébergé sur le fournisseur centralisé de services et qui assure : la déconnexion du PHIE lorsque le patient est admis dans la salle des urgences et/ou à l'hôpital ; le personnel de la salle des urgences et/ou de l'hôpital délivrant un diagnostic et/ou administrant des soins au patient ; le personnel de la salle des urgences et/ou de l'hôpital enregistrant tous les services de soins médicaux d'urgence qui sont prodigués au patient afin de créer un EHR mis à jour ; et un troisième moyen de rehaussement de serveur, qui est hébergé sur le fournisseur centralisé de services et qui assure : la constitution d'un sous-réseau privé entre le EHIE et un dispositif de moyen d'archivage de patient en permettant une liaison de communication entre le fournisseur centralisé de services et le dispositif de moyen d'archivage de patient, dans lequel un PHR est situé sur le dispositif de moyen d'archivage de patient ; la comparaison du EHR mis à jour et du PHR afin d'identifier que le EHR mis à jour dispose de données plus récentes ; la synchronisation du EHR mis à jour et du PHR de telle sorte que les deux EHR contiennent les données plus récentes ; et la mise à jour d'un registre qui est associé au EHR et au PHR afin d'enregistrer la transaction de synchronisation du EHR et du PHR.

14. Procédé selon la revendication 13, dans lequel le premier moyen de rehaussement de serveur, qui est hébergé sur le fournisseur centralisé de services, assure en outre : le fait d'aviser par notification un ou plusieurs contacts du service des urgences par courrier électronique et/ou au moyen d'un système d'envoi de messages courts.

15. Procédé selon la revendication 5, comprenant : la fourniture d'un système d'échange d'informations personnelles concernant la santé (PHIE) qui comprend un enregistrement personnel concernant la santé qui est stocké sur un moyen d'archivage personnel et au moins sur un moniteur de patient ; la collecte par un moniteur de patient de données biométriques et/ou médicales en temps réel à partir d'un patient ; la délivrance des données biométriques et/ou médicales en temps réel en provenance d'un patient à un fournisseur centralisé de services ; et un moyen de rehaussement de serveur, qui est hébergé sur le fournisseur centralisé de services et qui assure : la réalisation d'un traitement, d'une analyse statistique et du formatage des données biométriques et médicales en temps réel du patient afin de produire des données de patient traitées ; et la réalisation de l'ajout des données de patient traitées au niveau du PHR, dans lequel le moyen de rehaussement de serveur, qui est hébergé sur le fournisseur centralisé de services, assure en outre : la constitution d'un sous-réseau privé entre le dispositif de moyen d'archivage de patient et un moyen d'archivage de données standardisées concernant la santé en permettant une liaison de communication entre le fournisseur centralisé de services et un moyen d'archivage de données standardisées concernant la santé ; l'extraction de données de référence à partir du moyen d'archivage de données standardisées concernant la santé ; la comparaison des données de patient traitées avec les données de référence afin d'identifier des anomalies éventuelles en relation avec la santé qui sont présentées par le patient afin de produire un rapport de référence ; et la fourniture du rapport de référence pour son ajout au niveau du PHR.
